# EUROPEAN PATENT APPLICATION

(11) **EP 2 269 580 A2**
(43) Date of publication of application: **05.01.2011**
(21) Application number: 10174312.8
(22) Date of filing: 06.09.2005
(51) Int. Cl.: A61K 9/16, A61K 31/4745, A61P 35/04

(54) **Compositions comprising camptothecins in microspheres**

(30) Priority: 07.09.2004 EP 04255411
(62) Divisional of application: 05777645.2
(71) Applicant: Biocompatibles UK Limited, Farnham Surrey GU9 8QL (GB)
(72) Inventor: Lewis, Andrew Lennard, Farnham, surrey GU9 8QL (GB); Stratford, Peter William, Farnham, Surrey GU9 8QL (GB); Gonzalez-Fajardo, Maria Victoria, Madrid (ES); Tang, Yiqing, Guildford, surrey GU9 8QL (GB)
(74) Representative: Jones, Helen M.M.

(57) **Abstract**

A composition comprises microspheres formed of water-insoluble water-swellable anionic polymer having swollen diameter more than 100 µm, and a cationic camptothecin compound, preferably irinotecan. The microspheres are preferably formed of crosslinked polyvinylalcohol, preferably of ethylenically unsaturated polyvinylalcohol macromer, crosslinked with anionic ethylenically unsaturated anionic comonomer. The compositions are used to treat hypervascular tumours for instance colorectal metastases of the liver.

## Description

The scope of the invention herein is the preparation and use of microspheres for embolisation in which the microspheres comprise a water-insoluble polymer and a therapeutic amount of a camptothecin, preferably irinotecan hydrochloride, for the chemoembolisation of a tumour.

Camptothecin (CPT) and its analogs are a new class of anticancer agents that have been identified over the past several years. Camptothecin exists in two forms depending on the pH: An active lactone form at pH below 5 and an inactive carboxylate form at basic or physiological neutral pH. The A ring of camptothecin is the left hand ring in the core portion the following structures.

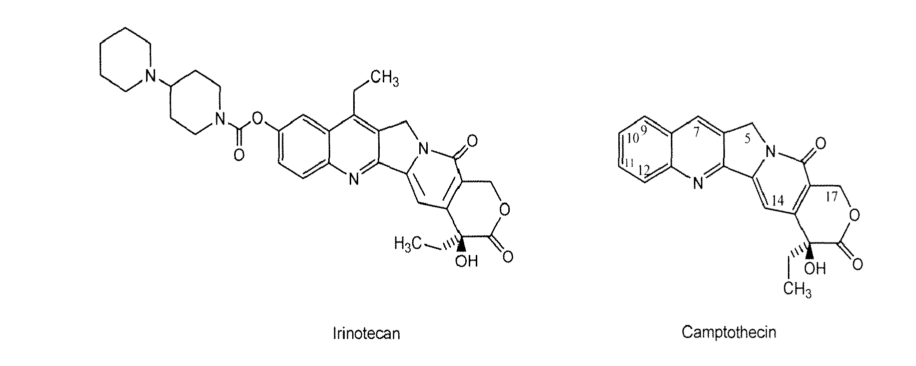

Irinotecan is a modified version of camptothecin that has been developed to improve the solubility and specificity of the drug. It is disclosed in US 4,604,463. Camptothecins interact specifically with the enzyme topoisomerase I which relieves torsional strain in DNA by inducing reversible single-strand breaks. Irinotecan and its active metabolite SN-38 bind to the topoisomerase I-DNA complex and prevent religation of these single-strand breaks. Current research suggests that the cytotoxicity of irinotecan is due to double-strand DNA damage produced during DNA synthesis when replication enzymes interact with the ternary complex formed by topoisomerase I, DNA, and either irinotecan or SN-38. Mammalian cells cannot efficiently repair these double-strand breaks.

Irinotecan in the form of its acid addition salt, eg. hydrochloride, serves as a somewhat water-soluble precursor of the lipophilic metabolite SN-38. SN-38 is formed from irinotecan by carboxylesterase-mediated cleavage of the carbamate bond between the camptothecin moiety and the dipiperidino side chain. SN-38 is approximately 1000 times as potent as irinotecan as an inhibitor of topoisomerase I purified from human and rodent tumour cell lines. In vitro cytotoxicity assays show that the potency of SN-38 relative to irinotecan varies from 2- to 2000-fold. However, the plasma area under the concentration versus time curve (AUC) values for SN-38 are 2% to 8% of those for irinotecan as SN-38 is 95% bound to plasma proteins compared to approximately 50% bound to plasma proteins for irinotecan.

Irinotecan injection can induce both early and late forms of diarrhea that appear to be mediated by different mechanisms. Early diarrhea (occurring during or shortly after infusion of irinotecan) is cholinergic in nature. It is usually transient and only infrequently is severe. It may be accompanied by symptoms of rhinitis, increased salivation, miosis, lacrimation, diaphoresis, flushing, and intestinal hyperperistalsis that can cause abdominal cramping.

It is one of the drugs of choice for the treatment of colorectal cancer and metastases of the liver (CRM). The drug is administered intravenously, usually in combination with other therapeutics. Others have used microparticles as a means of delivering the drug intravenously; in these cases the microparticles need to be small enough to avoid blocking blood vessels (Evaluation of camptothecin microspheres in cancer therapy. Tong, Wenkai. Avail. UMI, Order No. DA3061801. (2002), 214 pp. From: Diss. Abstr. Int., B 2003, 63(8), 3730; Injectable pharmaceutical composition comprising microparticles or microdroplets of camptothecin. Sands, Howard; Mishra, Awadhesh. (Supergen, Inc., USA; Rtp Pharma, Inc.). PCT Int. Appl. (2002), 103 pp.)

Poly(lactide-co-glycolide) (PLGA) microspheres have been considered good delivery vehicles for CPT because of acidic microenvironment formed through PLGA degradation (Evaluation of PLGA Microspheres as Delivery System for Antitumor Agent-Camptothecin. Tong, Wenkai; Wang, Lejun; D'Souza, Martin J. Drug Development and Industrial Pharmacy (2003), 29(7), 745-756) and Poly (D, L-lactic-co-glycolic acid) microspheres for sustained delivery and stabilization of camptothecin, Ertl, B., et al., J. Contr. Rel. 1999, 61, 305-317. Camptothecin or its derivatives are enclosed in polymers to give anticancer controlled-release microspheres with an average diameter of 2-70 µm. (Controlled-release microspheres containing antitumor agents. Machida, Masaaki; Onishi, Hiroshi; Morikawa, Akinobu; Machida, Ryoji; Kurita, Akinari. Jpn. Kokai Tokkyo Koho (2002), 7 pp.). Particles of this size are generally used for intravenous delivery, but can also be used as implants or be directly injected at a tumour site, e.g. during surgery. (Camptothecin Delivery Methods Hatefi, A. et al., Pharm. Res. 2002, 19(10) 1389-1399).

Others have investigated the effect of the polymer-drug interaction on the surface morphology of polymer microspheres and in vitro release properties. Polylactide microspheres enclosing Irinotecan hydrochloride (CPT) were prepared by the solvent evaporation method of the O/O emulsion system in order to control the concentration of drugs in living organisms. The mean diameter of the polylactide microspheres was kept at approximately 50 µm while varying the content of CPT (Surface morphology change of polylactide microspheres enclosing Irinotecan hydrochloride and its effect on release properties. Yoshizawa, Hidekazu; Nishino, Satoru; Natsugoe, Shoji; Aiko, Takashi; Kitamura, Yoshiro. Journal of Chemical Engineering of Japan (2003), 36(10), 1206-1211.).

Another study of delivery of a camptothecin derivative (1 0-hydroxy camptothecin) from degradable poly(lactide-co-glycolide) uses an emulsion of methylene chloride-polymer solution in water. The drug is added in the emulsion as a solution in DMF. The microspheres have average particle sizes in the range 27-82 µm. The intent is that the microspheres circulate and release drug over a period of weeks. Although there is a suggestion that encapsulated camptothecins might be useful for embolising hepatic tumours there is no indication how this may be achieved. (Stabilization of 10-hydroxycamptothecin in Poly(lactide-co-glycolide)microsphere delivery vehicles Shenderova, A. et al., Pharm. Res. 1997, 14(10) 1406-1414).

Biodegradable microspheres have been used to deliver the drug directly to the tumour by direct injection into the tumour mass (Use of biodegradable microspheres for the delivery of an anticancer agent in the treatment of glioblastoma. Faisant, Nathalie; Benoit, Jean-Pierre; Meinei, Philippe. WO-A-0069413. The microspheres are formed of polyglycolide and have average diameter 48µm.

The incorporation of the drug into microspheres has been shown to prolong the lifetime of the drug in the circulation (Pharmacokinetics of prolonged-release CPT-11-loaded microspheres in rats. Machida, Y.; Onishi, H.; Kurita, A.; Hata, H.; Morikawa, A.; Machida, Y. Journal of Controlled Release (2000), 66(2-3), 159-175.) CPT-11-contg. microspheres composed of poly(DL-lactic acid) or poly(DL-lactic acid-co-glycolic acid) copolymers were prepared by an oil-in-water evaporation method. The size and shape of the microspheres were examined, and the drug release rates were analyzed from the in vitro release profiles. CPT-11 aq. solution was i.v. or i.p. injected at 10 mg/kg, and microspheres were i.p. administered at 50 mg eq CPT-11/kg in rats. The microspheres had an average diameter of around 10 µm and their shape was spherical.

Others have attempted to target the microspheres by use of external magnetic fields (In vivo evaluation of camptothecin microspheres for targeted drug delivery. Sonavaria, Vandana J.; Jambhekar, Sunil; Maher, Timothy. Proceedings of the International Symposium on Controlled Release of Bioactive Materials (1994), 21 ST 194-5.) Magnetically responsive albumin microspheres containing camptothecin can be reliably targeted to the desired site in a rat model. In addition, the targeted microspheres remained localized for many hours after removal of the magnetic field suggesting that the microspheres were engulfed within the cells, and the drug released at the site. Presumably the microspheres are very small, probably about 1 µm.

One method for the palliative treatment of colorectal metastases of the liver is by chemoembolisation. In one procedure, an active pharmaceutical is introduced directly into the artery feeding the tumour via a catheter, followed by the introduction of embolic agent to stop or slow the flow into the diseased segment, hence reducing washout of the drug. There is no gold-standard method adopted and the therapeutics used are varied and include but are not limited to 5-FU, mitomycin C or mixtures of cisplatin, adriamycin and mitomycin (CAM) amongst others. Unusually, although irinotecan is a choice systemic treatment, it has not been adopted for widespread chemoembolisation. Only one recent study in rats has specifically combined irinotecan and embolisation by a method in which a solution of drug and a suspension of embolic starch microspheres was introduced into the hepatic artery. (Chemoembolization of rat liver metastasis with irinotecan and quantification of tumour cell reduction. Saenger Jan; Leible Maike; Seelig Matthias H; Berger Martin R. Journal of cancer research and clinical oncology (Germany) Apr 2004, 130 (4) p203-10.) The method used does not involve association of the drug with the polymer of the embolic material and hence no control of release of the drug. The starch microspheres merely slow the flow through the vessels and degrade within a period of less than about an hour.

WU, S.J., An Experimental study of the basic properties of drug microsphere and target treatment of rats with liver tumour, Zhonghua-Waike Za Zhi Apr 1990 28(4) 241-243, describes hepatic artery embolisation with camptothecin-albumin microspheres. The tumours were necrosed and tissue damage by tumour reversed with the microspheres.

There has been one clinical study on super-selective camptothecin microsphere's embolisation of internal iliac artery for bladder carcinoma (Xu A; Wang X; Yu M. Department of Urology, General Hospital of PLA, Beijing 100853, China. Zhonghua yi xue za zhi (China) May 2000, 80 (5) p358-9.) The nature of the microsphere was not specified. The size was about 200µm diameter. The authors evaluated the efficacy of camptothecin microsphere's embolisation of the internal iliac artery for bladder carcinoma. Eighteen patients with inoperable and advanced bladder carcinoma were treated with camptothecin microsphere's super-selective embolisation of the internal iliac artery. Tumour size was reduced significantly, and tumour cells were damaged to various degrees in 17 patients. Adverse effects were not found. They concluded that camptothecin microsphere embolisation of the internal iliac artery is a safe and effective therapy for inoperable and advanced bladder carcinoma.

According to the present invention there is provided a new use of microspheres comprising a water-insoluble water swellable polymer which is anionically charged at pH7 and electrostatically associated with the polymer in releasable form, a cationically charged camptothecin compound in the manufacture of a composition for use in a method of treatment in which the composition is introduced into a blood vessel and the microspheres form an embolus in the blood vessel in which the particles have sizes when equilibrated in water at 37°C, in the range 100 to 1500 µm in which method the camptothecin compound is released from the embolus.

The method of treatment is generally for therapy of a solid tumor. In the invention the microspheres have diameters when equilibrated with water at room temperature of more than 100 µm. Thus preferably substantially none of the microspheres have size of less than 100 µm. The sizes may be up to 200 µm, preferably up to 1500 µm. The diameter is preferably determined by measurement of the microsphere size prior to loading with the campothecin compound. Although the microspheres are preferably substantially spherical, they may be spheroidal or even less regular in shape. In the following description we refer to microspheres and particles inter changeably. The diameter of a non-spherical particle is its largest diameter.

The camptothecin compound is preferably at least sparingly water-soluble, for instance soluble to a concentration of at least 0.001g/l in water at room temperature preferably more than 0.002g/l more preferably more than 0.01 g/l. It is preferred that the camptothecin compound is cationically charged at pH7. The cationic group may be a primary amine group, but is preferably a secondary, tertiary or quaternary amine group.

One family of suitable compounds has the general formula I 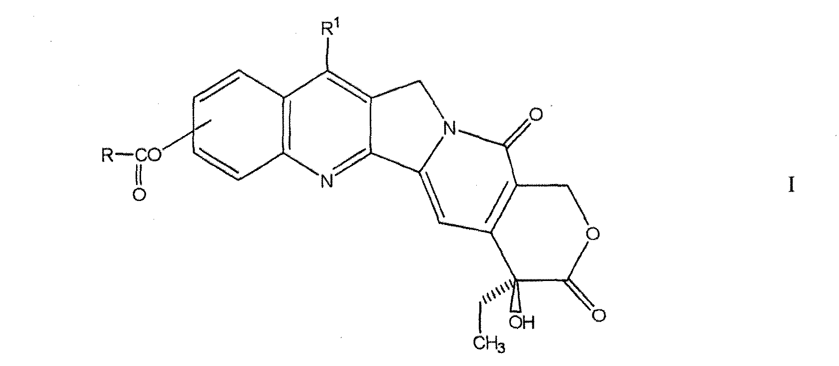 in which R¹ is H, lower (C₁₋₆) alkyl, optionally substituted by a hydroxyl, amine, alkoxy, halogen, acyl or acyloxy group or halogen; and

R is chlorine or NR²R³ where R² and R³ are the same or different and each represents a hydrogen atom, a substituted or unsubstituted C₁₋₄ alkyl group or a substituted or unsubstituted carbocyclic or heterocyclic group, or R² and R³ together with the nitrogen atom to which they are attached from a optionally substituted heterocyclic ring which may be interrupted by -O-, -S-or >NR⁴ in which R⁴ is a hydrogen atom, a substituted or unsubstituted C₁₋₄ alkyl group or a substituted or unsubstituted phenyl group;

and wherein the grouping -O-CO-R is bonded to a carbon atom located in any of the 9, 10 or 11 positions in the A ring of the camptothecin compound, including salts thereof.

It is preferred for the grouping -O-CO-R to be joined at the 10 position.

R¹ is preferably C₁₋₄ alkyl, most preferably ethyl, and m is preferably 1.

A halogen atom R is, for instance, F, Cl, Br or preferably F or Cl.

R¹ to R⁴ may be methyl, ethyl, propyl, isopropyl, in-butyl, isobutyl and t-butyl, preferably methyl.

Substituents in R and R¹ are preferably selected from halogen atoms, hydroxy, C₁₋₄ alkoxy, phenoxy, COOR⁶, SO₃R⁶ and PO₃(R⁶)₂, aryl, 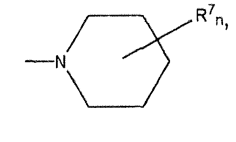 NR⁸R⁹ and CONR⁸R⁹, QAOR⁵, QANR⁸R⁹ and QAQR⁵ in
which R⁵ is C₁₋₄ alkyl or aryl; R⁶ is hydrogen, halogen C₁₋₄ alkyl or C₁₋₄ alkoxy; R⁷ is hydrogen, halogen or C₁₋₄ alkyl; R⁸ and R⁹ are the same or different and each is H, or C₁₋₄ alkyl or R⁸ and R⁹ together represent C₃₋₆ alkanediyl;

Q is OCO, or -COO- and A is C₂₋₄ alkanediyl.

Preferably R is NR³ R³ where R² and R³ together with the nitrogen atom form a 5 or 6 membered ring, preferably a saturated ring, with optional substituents. A subsituent is preferably -NR⁸R⁹. In such a substituent R⁸ and R⁹ preferably together are C₄₋₅ alkanediyl. Such groups are basic and tend to be cationically charged at pH7. Most preferably R is

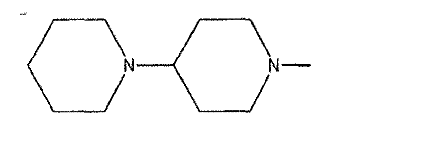

Another family of suitable compounds has the general formula II 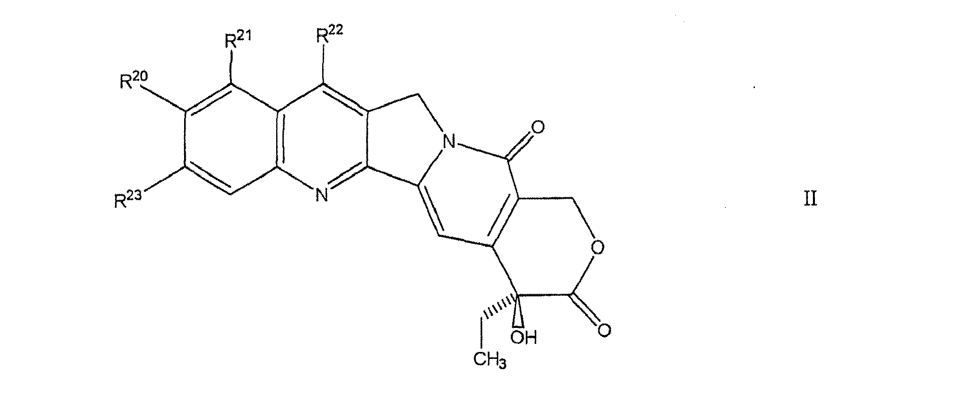 in which R²⁰ and R²³ are each hydroxy or hydrogen or together are CH₂OCH₂;

one of R²¹ and R²² is H and the other is CH₂NR²⁴R²⁵ where R²³ and R²⁴ are the same or different and each represents a hydrogen atom, a substituted or unsubstituted C₁₋₄ alkyl group or a substituted or unsubstituted carbocyclic or heterocyclic group, or R²³ and R²⁴ together with the nitrogen atom to which they are attached from a optionally substituted heterocyclic ring which may be interrupted by -O-, -S- or >NR⁴ in which R⁴ is a hydrogen atom, a substituted or unsubstituted C₁₋₄ alkyl group or a substituted or unsubstituted phenyl group; including salts and quaternary derivatives thereof. One example of a suitable compound of this claim is topotecan, in which R²⁰ is hydroxyl, R²² and R²³ are hydrogen, R²¹ is CH₂NR²⁴R²⁵ and R²⁴ and R²⁵ are both methyl.

The polymer is a water-insoluble material. Although it may be biodegradable, so that drug may be released substantially by erosion of polymer matrix to release drug from the surface, preferably the polymer is substantially biostable (ie non-biodegradable).

The polymer is water-swellable. Water-swellable polymer useful in the invention preferably has a equilibrium water content, when swollen in water at 37°C, measured by gravimetric analysis, in the range of 40 to 99 wt%, preferably 75 to 95%.

In the preferred embodiment of the invention, the composition which is administered to a patient in need of embolisation therapy, is in the form of a suspension of particles of water-swollen water-insoluble polymer in a liquid carrier. Preferably the particles are graded into calibrated size ranges for accurate embolisation of vessels. The particles preferably have sizes when equilibrated in water at 37°C, in the range 100 to 1500 µm, more preferably in the range 100 to 1200 µm. The calibrated ranges may comprise particles having diameters with a bandwidth of about 100 to 300 µm. The size ranges may be for instance 100 to 300 µm, 300 to 500 µm, 500 to 700 µm, 700 to 900 µm and 900 to 1200 µm. Preferably the particles are substantially spherical in shape. Such particles are referred to herein as microspheres.

Generally the polymer is covalently crosslinked, although it may be appropriate for the polymer to be ionically crosslinked, at least in part. Although it may be suitable to use polymers which are derived from natural sources, such as albumin, alginate, gelatin, starch, chitosan or collagen, all of which have been used as embolic agents, the polymer is preferably substantially free of naturally occurring polymer or derivatives. It is preferably formed by polymerising ethylenically unsaturated monomers in the presence of di- or higher-functional crosslinking monomers. The ethylenically unsaturated monomers may include an ionic (including zwitterionic) monomer.

Copolymers of hydroxyethyl methacrylate, acrylic acid and cross-linking monomer, such as ethylene glycol dimethacrylate or methylene bisacrylamide, as used for etafilcon A based contact lenses may be used. Copolymers of N-acrylayl-2-amino-2-hydroxymethyl-propane-1,3-diol and N,N-bisacrylamide may also be used.

Other polymers are cross-linking styrenic polymers e.g. with ionic substituents, of the type used as separation media or as ion exchange media.

Another type of polymer which may be used to form the water-swellable water-insoluble matrix is polyvinyl alcohol crosslinked using aldehyde-type crosslinking agents such as glutaraldehyde. For such products, the polyvinyl alcohol (PVA) may be rendered ionic by providing pendant ionic groups by reacting a functional ionic group containing compound with the hydroxyl groups. Examples of suitable functional groups for reaction with the hydroxyl groups are acylating agents, such as carboxylic acids or derivatives thereof, or other acidic groups which may form esters.

The invention is of particular value where the polymer matrix is formed from a polyvinyl alcohol macromer, having more than one ethylenically unsaturated pendant group per molecule, by radical polymerisation of the ethylenic groups. Preferably the PVA macromer is copolymerised with ethylenically unsaturated monomers for instance including a nonionic and/or ionic monomer including anionic monomer.

The PVA macromer may be formed, for instance, by providing PVA polymer, of a suitable molecular weight such as in the range 1000 to 500,000 D, preferably 10,000 to 100,000 D, with pendant vinylic or acrylic groups. Pendant acrylic groups may be provided, for instance, by reacting acrylic or methacrylic acid with PVA to form ester linkages through some of the hydroxyl groups. Other methods for attaching vinylic groups capable of polymerisation onto polyvinyl alcohol are described in, for instance, US 4,978,713 and, preferably, US 5,508,317 and 5,583,163. Thus the preferred macromer comprises a backbone of polyvinyl alcohol to which is linked, via a cyclic acetal linkage, an (alk)acrylaminoalkyl moiety. Example 1 describes the synthesis of an example of such a macromer known by the approved named nelfilcon B. Preferably the PVA macromers have about 2 to 20 pendant ethylenic groups per molecule, for instance 5 to 10.

Where PVA macromers are copolymerised with ethylenically unsaturated monomers including an ionic monomer, the ionic monomer preferably has the general formula II 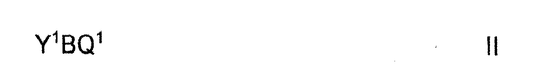 in which Y¹ is selected from 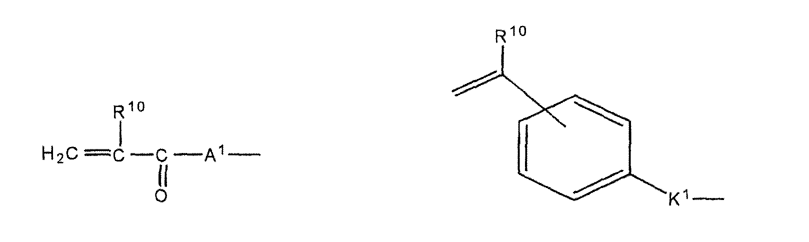 CH₂=C(R¹⁰)-CH₂-O-, CH₂=C(R¹⁰)-CH₂ OC(O)-, CH₂=C(R¹⁰)OC(O)-, CH₂=C(R¹⁰)-O-, CH₂=C(R¹⁰)CH₂OC(O)N(R¹¹)-, R¹²OOCCR¹⁰=CR¹⁰C(O)-O-, R¹⁰CH=CHC(O)O-, R¹⁰CH=C(COOR¹²)CH₂-C(O)-O-, 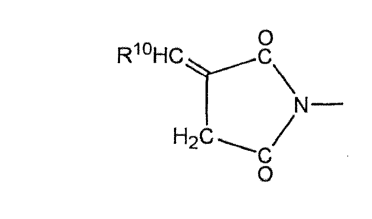 and 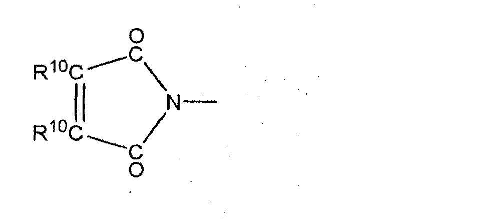 wherein:
R¹⁰ is hydrogen or a C₁-C₄ alkyl group;
R¹¹ is hydrogen or a C₁-C₄ alkyl group;
R¹² is hydrogen or a C₁₋₄ alkyl group or BQ¹ where B and Q¹ are as defined below;
A¹ is -O- or -NR¹¹ -;
K¹ is a group -(CH₂),OC(O)-, -(CH₂),C(O)O-, -(CH₂),OC(O)O-, -(CH₂)ᵣNR¹³-, -(CH₂)ᵣNR¹³C(O)-, -(CH₂)ᵣC(O)NR¹³-, -(CH₂)ᵣNR¹³C(O)O-, -(CH₂)ᵣOC(O)NR¹³-, -(CH₂)ᵣNR¹³C(O)NR¹³- (in which the groups R¹³ are the same or different), -(CH₂)ᵣO-, -(CH₂)ᵣSO₃ -, or, optionally in combination with B, a valence bond and r is from 1 to 12 and R¹³ is hydrogen or a C₁-C₄ alkyl group;
B is a straight or branched alkanediyl, oxaalkylene, alkanediyloxaalkanediyl, or alkanediyloligo(oxaalkanediyl) chain optionally containing one or more fluorine atoms up to and including perfluorinated chains or, if Q¹ or Y¹ contains a terminal carbon atom bonded to B a valence bond; and
Q¹ is an ionic group.

Such a compound including an anionic group Q¹ is preferably included.

An anionic group Q¹ may be, for instance, a carboxylate, carbonate, sulphonate, sulphate, nitrate, phosphonate or phosphate group. The monomer may be polymerised as the free acid or in salt form. Preferably the pKₐ of the conjugate acid is less than 5.

A suitable cationic group Q¹ is preferably a group N⁺R¹⁴₃, P⁺R¹⁵₃ or S⁺R¹⁵₂ in which the groups R¹⁴ are the same or different and are each hydrogen, C₁₋₄-alkyl or aryl (preferably phenyl) or two of the groups R¹⁴ together with the heteroatom to which they are attached from a saturated or unsaturated heterocyclic ring containing from 5 to 7 atoms the groups R¹⁵ are each OR¹⁴ or R¹⁴. Preferably the cationic group is permanently cationic, that is each R¹⁴ is other than hydrogen. Preferably a cationic group Q is N⁺R¹⁴₃ in which each R¹⁴ is C₁₋₄-alkyl, preferably methyl.

A zwitterionic group Q¹ may have an overall charge, for instance by having a divalent centre of anionic charge and monovalent centre of cationic charge or vice-versa or by having two centres of cationic charge and one centre of anionic charge or vice-versa. Preferably, however, the zwitterion has no overall charge and most preferably has a centre of monovalent cationic charge and a centre of monovalent anionic charge.

Examples of zwitterionic groups which may be used as Q in the present invention are disclosed in WO-A-0029481.

Where the ethylenically unsaturated monomer includes zwitterionic monomer, for instance, this may increase the hydrophilicity, lubricity, biocompatibility and/or haemocompatibility of the particles. Suitable zwitterionic monomers are described in our earlier publications WO-A-9207885, WO-A-9416748, WO-A-9416749 and WO-A-9520407. Preferably a zwitterionic monomer is 2-methacryloyloxy-2'-trimethylammonium ethyl phosphate inner salt (MPC).

In the monomer of general formula I preferably Y¹ is a group CH²=CR¹⁰COA- in which R¹⁰ is H or methyl, preferably methyl, and in which A¹ is preferably NH. B is preferably an alkanediyl group of 1 to 12, preferably 2 to 6 carbon atoms. Such monomers are acrylic monomers.

There may be included in the ethylenically unsaturated monomer diluent monomer, for instance non-ionic monomer. Such a monomer may be useful to control the pKₐ of the acid groups, to control the hydrophilicity or hydrophobicity of the product, to provide hydrophobic regions in the polymer, or merely to act as inert diluent. Examples of non-ionic diluent monomer are, for instance, alkyl (alk) acrylates and (alk) acrylamides, especially such compounds having alkyl groups with 1 to 12 carbon atoms, hydroxy, and dihydroxy-substituted alkyl(alk) acrylates and -(alk) acrylamides, vinyl lactams, styrene and other aromatic monomers.

In the polymer matrix, the level of anion is preferably in the range 0.1 to 10 meq g⁻¹, preferably at least 1.0 meq g⁻¹. Preferred anions are derived from strong acids, such as sulphates sulphonats, phosphates and phosphonates.

Where PVA macromer is copolymerised with other ethylenically unsaturated monomers, the weight ratio of PVA macromer to other monomer is preferably in the range of 50:1 to 1:5, more preferably in the range 20:1 to 1:2. In the ethylenically unsaturated monomer the anionic monomer is preferably present in an amount in the range 10 to100 mole%, preferably at least 25 mole%.

The crosslinked polymer may be formed as such in particulate form, for instance by polymerising in droplets of monomer in a dispersed phase in a continuous immiscible carrier. Examples of suitable water-in-oil polymerisations to produce particles having the desired size, when swollen, are known. For instance US 4,224,427 describes processes for forming uniform spherical beads (microspheres) of up to 5 mm in diameter, by dispersing water-soluble monomers into a continuous solvent phase, in a presence of suspending agents. Stabilisers and surfactants may be present to provide control over the size of the dispersed phase particles. After polymerisation, the crosslinked microspheres are recovered by known means, and washed and optionally sterilised. Preferably the particles eg microspheres, are swollen in an aqueous liquid, and classified according to their size.

The campethecin compound is associated with the polymer preferably so as to allow controlled release of the agent over a period. This period may be from several minutes to weeks, preferably at least up to a few days, preferably up to 72 hours. The agent is electrostatically bonded to the polymer. The presence of anionic groups in the polymer allows control of release of cationically charged camptothecin active.

The pharmaceutical active may be incorporated into the polymer matrix by a variety of techniques. In one method, the active may be mixed with a precursor of the polymer, for instance a monomer or macromer mixture or a cross-linkable polymer and cross-linker mixture, prior to polymerising or crosslinking. Alternatively, the active may be loaded into the polymer after it has been crosslinked. For instance, particulate dried polymer may be swollen in a solution of active, preferably in water or in an alcohol such as ethanol, optionally with subsequent removal of non-absorbed agent and/or evaporation of solvent. A solution of the active, in an organic solvent such as an alcohol, or, more preferably, in water, may be sprayed onto a moving bed of particles, whereby drug is absorbed into the body of the particles with simultaneous solvent removal. Most conveniently, we have found that it is possible merely to contact swollen particles suspended in a continuous liquid vehicle, such as water, with an aqueous alcoholic solution of drug, over a period, whereby drug becomes absorbed into the body of the particles. Techniques to fix the drug in the particles may increase loading levels, for instance, precipitation by shifting the pH of the loading suspension to a value at which the active is in a relatively insoluble form. The swelling vehicle may subsequently be removed or, conveniently, may be retained with the particles as part of the product for subsequent use as an embolic agent or the swollen particles may be used in swollen form in the form of a slurry, i.e. without any or much liquid outside the swollen particles. Alternatively, the suspension of particles can be removed from any remaining drug loading solution and the particles dried by any of the classical techniques employed to dry pharmaceutical-based products. This could include, but is not limited to, air drying at room or elevated temperatures or under reduced pressure or vacuum; classical freeze-drying; atmospheric pressure-freeze drying; solution enhanced dispersion of supercritical fluids (SEDS). Alternatively the drug-loaded microspheres may be dehydrated using an organic solvent to replace water in a series of steps, followed by evaporation of the more volatile organic solvent. A solvent should be selected which is a non-solvent for the drug.

In brief, a typical classical freeze-drying process might proceed as follows: the sample is aliquoted into partially stoppered glass vials, which are placed on a cooled, temperature controlled shelf within the freeze dryer. The shelf temperature is reduced and the sample is frozen to a uniform, defined temperature. After complete freezing, the pressure in the dryer is lowered to a defined pressure to initiate primary drying. During the primary drying, water vapour is progressively removed from the frozen mass by sublimation whilst the shelf temperature is controlled at a constant, low temperature. Secondary drying is initiated by increasing the shelf temperature and reducing the chamber pressure further so that water absorbed to the semi-dried mass can be removed until the residual water content decreases to the desired level. The vials can be sealed, *in situ,* under a protective atmosphere if required.

Atmospheric pressure freeze-drying is accomplished by rapidly circulating very dry air over a frozen product. In comparison with the classical freeze-drying process, freeze-drying without a vacuum has a number of advantages. The circulating dry gas provides improved heat and mass transfer from the frozen sample, in the same way as washing dries quicker on a windy day. Most work in this area is concerned with food production, and it has been observed that there is an increased retention of volatile aromatic compounds, the potential benefits of this to the drying of biologicals is yet to be determined. Of particular interest is the fact that by using atmospheric spray-drying processes, instead of a cake, a fine, free-flowing powder is obtained. Particles can be obtained which have submicron diameters, this is ten-fold smaller than can be generally obtained by milling. The particulate nature, with its high surface area results in an easily rehydratable product, currently the fine control over particle size required for inhalable and transdermal applications is not possible, however there is potential in this area.

Although the composition may be made up from polymer and camptothecin compound immediately before administration, it is preferred that the composition is preformed. Dried polymer-camptothecin particles may be hydrated immediately before use. Alternatively the composition which is supplied may be fully compounded and preferably comprises polymer particles with absorbed or absorbed camptothecin compound and imbibed water e.g physiological saline and extra-particulate liquid, for instance saline.

The level of camptothecin compound in the composition which is administered is preferable in the range 0.1 to 500mg per ml composition preferably 10 to 100mg per ml. Preferably the chemoembolisation method is repeated one is five times and for each dose the amount of camptothecin compound administered is in the range 0.1 to 100mg per ml, preferably 10 to 100mg per ml. The amount of composition administered in a normal embolisation is in the range 1 to 6ml. The total amount of camptothecin compound administered per dose is preferably in the range 10 to 1000mg, more preferably 50 to 250mg. Based on the release data as shown in the examples below, the inventors believe this will give therapeutically effective concentrations in the blood vessels at a tumor and that significant levels of intracellular delivery should take place whereby a therapeutic effect will be achieved. The adverse side effects of systemic camptothecin administration should be avoided.

The embolic compositions may be admixed in the normal manner for tumor embolisation. Thus the composition may be administered immediately before administration by the inventional radiologist, with imaging agents such as radiopaque agents. Additionally or alternatively the particles may be pre-loaded with radiopaque material in addition to the camptothecin compound. Thus the polymer and pharmaceutical active, provided as a preformed admixture, may be premixed with a radiopaque imaging agent in a syringe used as the reservoir for the delivery device. The composition may be administered, for instance, from a microcatheter device into the appropriate artery. Selection of suitable particle size range, dependent upon the eventual site of embolisation, may be made in the normal way by the interventional radiologist.

The invention is expected to be a benefit in the treatment of primary and secondary tumours which are hypervascular, and hence embolisable, such as primary liver cancer (hepatocellular carcinoma, HCC), metastases to the liver (colorectal, breast, endocrine), and renal, bone, breast, bladder, prostate, colon and lung tumours.

There is also disclosed herein:
A. A composition comprising microspheres comprising a water-insoluble water-swellable polymer which is anionically charged at pH7 and electrostatically associated with the polymer in releasable form, a cationically charged camptothecin compound in which the microspheres have diameters when equilibrated with water at room temperature of more than 100µm.
B. A composition according to A in which the camptothecin compound has the general formula I 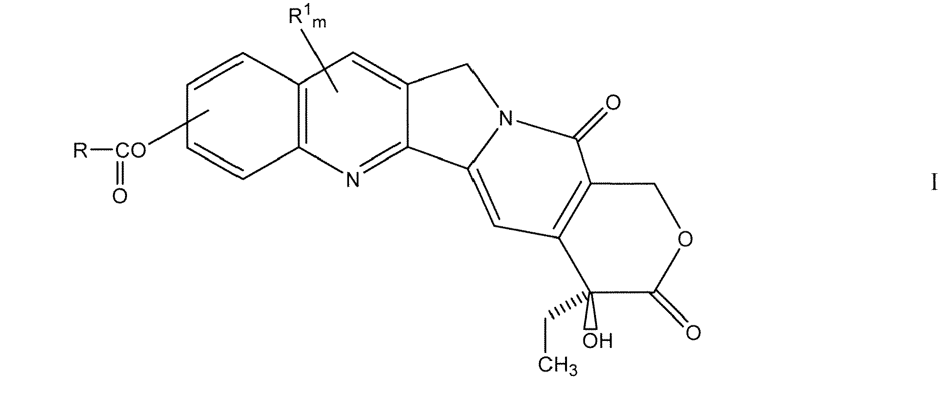 in which R¹ is H, lower (C₁₋₆) alkyl, optionally substituted by a hydroxyl amine, alkoxy, halogen, acyl or acyloxy group or halogen; and
   R is chlorine or NR²R³ where R² and R³ are the same or different and each represents a hydrogen atom, a substituted or unsubstituted C₁₋₄ alkyl group or a substituted or unsubstituted carbocyclic or heterocyclic group, or R² and R³ together with the nitrogen atom to which they are attached from a optionally substituted heterocyclic ring which may be interrupted by -O-, -S- or >NR⁴ in which R⁴ is a hydrogen atom, a substituted or unsubstituted C₁₋₄ alkyl group or a substituted or unsubstituted phenyl group;
   and wherein the grouping -0-CO-R is bonded to a carbon atom located in any of the 9, 10 or 11 positions in the A ring of the camptothecin compound, including salts thereof.
C. A composition according to B in which R is NR²R³ in which R² and R³ together with the nitrogen atom form a optionally substituted heterocyclic ring.
D. A composition according to C in which R is 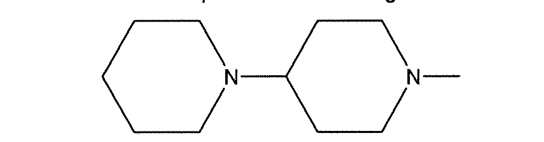
E. A composition according to any of B to D in which RCOO- is substituted at the 10 position.
F. A composition according to any of B to E in which R¹ is ethyl and m is 1.
G. A composition according to any of B to F in which the polymer is crosslinked polyvinylalcohol.
H. A composition according to G in which the polymer have sizes when equilibrated in water at 37°C, in the range 100 to 1500 µm, more preferably in the range 200 to 1200 µm.
I. A composition according to H in which the PVA macromer is copolymerised with ethylenically unsaturated monomer.
J. A composition according to I in which the monomer includes ionic monomer having the general formula II 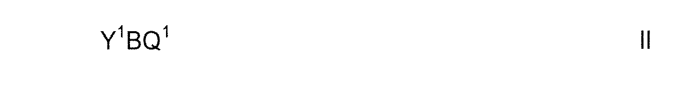 in which Y¹ is selected from 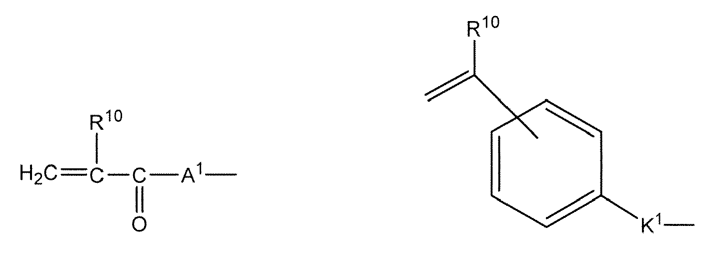 CH₂=C(R¹⁰)-CH₂-O-, CH₂=C(R¹⁰)-CH₂ OC(O)-, CH₂=C(R¹⁰)OC(O)-, CH₂=C(R¹⁰)-O-, CH₂=C(R¹⁰)CH₂OC(O)N(R¹¹)-, R¹²OOCCR¹⁰=CR¹⁰C(O)-O-, R¹⁰CH=CHC(O)O-, R¹⁰CH=C(COOR¹²)CH₂-C(O)-O-, 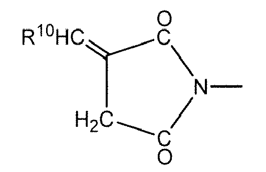 and 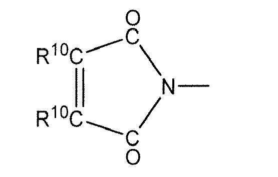 wherein:
   R¹⁰ is hydrogen or a C₁-C₄ alkyl group;
   R¹¹ is hydrogen or a C₁-C₄ alkyl group;
   R¹² is hydrogen or a C₁₋₄ alkyl group or BQ¹ where B and Q¹ are as defined below;
   A¹ is -O- or -NR¹¹ -;
   K¹ is a group -(CH₂)ᵣOC(O)-, -(CH₂)ᵣC(O)O-, - (CH₂)ᵣOC(O)O-, -(CH₂)ᵣNR¹³-, -(CH₂)ᵣNR¹³C(O)-, -(CH₂)ᵣC(O)NR¹³-, -(CH₂)ᵣNR¹³C(O)O-, -(CH₂)ᵣOC(O)NR¹³-, - (CH₂)ᵣNR¹³C(O)NR¹³- (in which the groups R¹³ are the same or different), -(CH₂)ᵣO--(CH₂)ᵣSO₃ -, or, optionally in combination with B, a valence bond and r is from 1 to 12 and R¹³ is hydrogen or a C₁-C₄ alkyl group;
   B is a straight or branched alkanediyl, oxaalkylene, alkanediyloxaalkanediyl, or alkanediyloligo(oxaalkanediyl) chain optionally containing one or more fluorine atoms up to and including perfluorinated chains or, if Q¹ or Y¹ contains a terminal carbon atom bonded to B a valence bond; and
   Q¹ is an anionic group.
K. A composition according to J in which Q¹ is a carboxylate, carbonate, sulphonate, sulphate, nitrate, phosphonate or phosphate group.
L. A composition according to J or K in which Y¹ is a group CH₂=CR¹⁰COA-in which R¹⁰ is H or methyl, preferably methyl, and in which A¹ is preferably NH and B is an alkanediyl group of 2 to 6 carbon atoms.
M. A composition according to any of B to L which additionally comprises a liquid suspending agent, preferably a pharmaceutically acceptable liquid more preferably an aqueous liquid, most preferably water.
N. A composition according to any of B to M which comprises an imaging agent preferably a radiopaque imaging agent.
O. A composition according to any of B to L which is substantially dry particulate form, preferably lyophilised form.

The invention is further illustrated in the following examples. Some of the results are shown in the accompanying figures, described in more detail in the examples, but briefly described as follows:
Figure 1 shows the loading of irinotecan from several different beads as described in example 1;
Figure 2 shows the elution from the beads loaded in example 1 , into phosphate buffered silane;
Figure 3 shows the elution profiles for irinotecan from the beads loaded in example 1 into water;
Figure 4 shows the loading capacity exemplified in example 2;
Figure 5 shows the change in size of beads as determined in example 3;
Figure 6 shows the effect of bead size and ionic group content on drug loading as exemplified in example 4;
Figure 7 shows the elution of irinotecan from gel spheres as described in example 6;
Figure 8 shows the results of example 7;
Figure 9 shows the chemiluminescence results of Example 9;
Figure 10 shows the number of tumour cells in livers after the trials in Example 9;
Figure 11 shows photographs of the livers of control and test rats after the trials in Example 9.

### Reference Example: Outline Method for the Preparation of Microspheres

### Nelfilcon B macromer synthesis:

The first stage of microsphere synthesis involves the preparation of Nelfilcon B - a polymerisable macromer from the widely used water soluble polymer PVA. Mowiol 8-88 polyvinyl alcohol) (PVA) powder (88% hydrolised, 12% acetate content, average molecular weight about 67,000D) (15Og) (Clariant, Charlotte, NC USA) is added to a 21 glass reaction vessel. With gentle stirring, 1000ml water is added and the stirring increased to 400rpm. To ensure complete dissolution of the PVA, the temperature is raised to 99 ± 9°C for 2-3 hours. On cooling to room temperature N- acryloylaminoacetaldehyde (NAAADA) (Ciba Vision, Germany) (2.49g or 0.104mmol/g of PVA) is mixed in to the PVA solution followed by the addition of concentrated hydrochloric acid (100ml) which catalyses the addition of the NAAADA to the PVA by transesterification. The reaction proceeds at room temperature for 6-7 hours then stopped by neutralisation to pH 7.4 using 2.5M sodium hydroxide solution. The resulting sodium chloride plus any unreacted NAAADA is removed by diafiltration (step 2).

### Diafiltration of macromer:

Diafiltration (tangential flow filtration) works by continuously circulating a feed solution to be purified (in this case nelfilcon B solution) across the surface of a membrane allowing the permeation of unwanted material (NaCl, NAAADA) which goes to waste whilst having a pore size small enough to prevent the passage of the retentate which remains in circulation.

Nelfilcon B diafiltration is performed using a stainless steel Pellicon 2 Mini holder stacked with 0.1m² cellulose membranes having a pore size witha molecular weight cut off of 3000 (Millipore Corporation, Bedford, MA USA). Mowiol 8-88 has a weight average molecular weight of 67000 and therefore has limited ability to permeate through the membranes.

The flask containing the macromer is furnished with a magnetic stirrer bar and placed on a stirrer plate. The solution is fed in to the diafiltration assembly via a Masterflex LS peristaltic pump fitted with an Easy Load II pump head and using LS24 class VI tubing. The Nelfilcon is circulated over the membranes at approximately 50psi to accelerate permeation. When the solution has been concentrated to about 1000ml the volume is kept constant by the addition of water at the same rate that the filtrate is being collected to waste until 6000ml extra has been added. Once achieved, the solution is concentrated to 20-23% solids with a viscosity of 1700-3400 cP at 25°C. Nelfilcon is characterised by GFC, NMR and viscosity.

### Microsphere Synthesis:

The spheres are synthesised by a method of suspension polymerisation in which an aqueous phase (nelfilcon B) is added to an organic phase (butyl acetate) where the phases are immiscible. By employing rapid mixing the aqueous phase can be dispersed to form droplets, the size and stability of which can be controlled by factors such as stirring rates, viscosity, ratio of aqueous/organic phase and the use of stabilisers and surfactants which influence the interfacial energy between the phases. Two series of microspheres are manufactured, a low AMPS and a higher AMPS series, the formulation of which are shown below.

| A | High AMPS: |
|---|---|
| Aqueous: | ca 21 % w/w Nelfilcon B solution (400±50g approx) |
| | ca 50% w/w 2-acrylamido-2-methylpropanesulphonate Na salt |
| | (140±1 Og) |
| | Purified water (137±30g) |
| | Potassium persulphate (5.22±0.1 g) |
| | Tetramethyl ethylene diamine TMEDA (6.4±0.1 ml) |
| Organic: | n-Butyl acetate (2.7+0.3L) 10% w/w cellulose acetate butyrate in |
| | ethyl acetate (46±0.5g) |
| | Purified water (19.0±0.5ml) |
| B | Low AMPS: |
| Aqueous: | a 21 % w/w Nelfilcon B solution (900±100g approx) |
| | ca 50% w/w 2-acryamido-2-methylpropanesulphonate Na salt |
| | (30.6±6g) |
| | Purified water (426±80g) |
| | Potassium persulphate (20.88±0.2g) |
| | TMEDA (25.6±0.5ml) |
| Organic: | n-Butyl acetate (2.2±0.3L) |
| | 10% w/w cellulose acetate butyrate (CAB) in ethyl acetate |
| | (92±1.0g) |
| | Purified water (16.7 +-0.5ml) |

A jacketed 4000ml reaction vessel is heated using a computer controlled bath (Julabo PN 9-300-650) with feedback sensors continually monitoring the reaction temperature.

The butyl acetate is added to the reactor at 25°C followed by the CAB solution and water. The system is purged with nitrogen for 15 minutes before the PVA macromer is added. Crosslinking of the dispersed PVA solution is initiated by the addition of TMEDA and raising the temperature to 55°C for three hours under nitrogen. Crosslinking occurs via a redox initiated polymerisation whereby the amino groups of the TMEDA react with the peroxide group of the potassium persulphate to generate radical species. These radicals then initiate polymerisation and crosslinking of the double bonds on the PVA and AMPS transforming the dispersed PVA-AMPS droplets into insoluble polymer microspheres. After cooling to 25°C the product is transferred to a filter reactor for purification where the butyl acetate is removed by filtration followed by:
- Wash with 2 x 300ml ethyl acetate to remove butyl acetate and CAB
- Equilibrate in ethyl acetate for 30mins then filtered
- Wash with 2 x 300 ml ethyl acetate under vacuum filtration
- Equilibrate in acetone for 30mins and filter to remove ethyl acetate, CAB and water
- Wash with 2 x 300ml acetone under vacuum filtration
- Equilibrate in acetone overnight
- Wash with 2 x 300ml acetone under vacuum
- Vacuum dry, 2hrs, 55°C to remove residual solvents.

### Dyeing:

This step is optional. It is generally unnecessary when drug is loaded with a coloured active (as this provides the colour) but in this it mentions there are advantages apparent from Example 8 below. When hydrated the microsphere contains about 90% (w/w) water and can be difficult to visualise. To aid visualisation in a clinical setting the spheres are dyed blue using reactive blue #4 dye (RB4). RB4 is a water soluble chlorotriazine dye which under alkaline conditions will react with the pendant hydroxyl groups on the PVA backbone generating a covalent ether linkage. The reaction is carried out at pH12 (NaOH) whereby the generated HCl will be neutralised resulting in NaCl.

Prior to dyeing, the spheres are fully re-hydrated and divided into 35g aliquots (treated individually). Dye solution is prepared by dissolving 0.8g RB4 in 2.5M NaOH solution (25ml) and water (15ml) then adding to the spheres in 21 of 80g/l⁻¹ saline. After mixing for 20mins the product is collected on a 32 µm sieve and rinsed to remove the bulk of the unreacted dye.

### Extraction:

An extensive extraction process is used to remove any unbound or non specifically adsorbed RB4. The protocol followed is as shown:
- Equilibrate in 21 water for 5mins. Collect on sieve and rinse. Repeat 5 times
- Equilibrate in 21 solution of 8OmM disodium hydrogen phosphate in 0.29% (w/w) saline. Heat to boiling for 30mins. Cool, collect on sieve and wash with 11 saline. Repeat twice more.
- Collect, wash on sieve the equilibrate in 21 water for 10mins.
- Collect and dehydrate in 11 acetone for 30mins.
- Combine all aliquots and equilibrate overnight in 21 acetone.

### Sieving:

The manufactured microsphere product ranges in size from 100 to 1200 microns and must undergo fractionation through a sieving process using a range of mesh sizes to obtain the nominal distributions listed below.
1. 100 - 300µm
2. 300 - 500µm
3. 500 - 700µm
4. 700 - 900µm
5. 900 - 1200µm

Prior to sieving, the spheres are vacuum dried to remove any solvent then equilibrated at 6O°C in water to fully re-hydrate. The spheres are sieved using a 316L stainless steel vortisieve unit (MM Industries, Salem Ohio) with 38 cm (15 in) stainless steel sieving trays with mesh sizes ranging from 32 to 1000µm. Filtered saline is recirculated through the unit to aid fractionation. Spheres collected in the 32micron sieve are discarded.

### Example 1: Loading & Elution of Irinotecan from Embolisation Beads The following microsphere ("Bead") products were tested:

1. High AMPS microsphere ("Gelsphere GS") (made as in 5 Example 1 ) particle size fraction 100 to 300µm, 500-700µm and 900-1200µm equilibrium water content 94%. (Invention)
2. Contour SE, a commercially available embolic product comprising non-ionic polyvinylalcohol microspheres particle size fraction 500-700 µm, equilibrium water content 40%. (reference)
3. Low AMPS microspheres ("BeadBlock - BB") made as in Example 1 ) above particle size range 100 to 300 µm, equilibrium water content 90%. (Invention)
4. Embosphere - a commercially available embolic agent comprising particles of N∼acryloyl-2-amino-2-hydroxy methyl- propane-1 ,3-diol-co-N,N-bisacrylamide) copolymer cross-linked with gelatin and glutaraldehyde having particle size ranges 100-300 and 500 to 700 µm. This polymer at neutral pH has a net positive charge from the gelatin content. (FR-A-7723223). The equilibrium water content is 91 %. (Reference)
5. Amberlite ira400 (strongly basic gel type resine, quaternary ammonium functionality, average size =510 µm, WC= 52.44%). (Reference)
6. Amberlyst 36 (wet), very strongly acidic, sulfonic acid functionality, hydrogen form , average size=667 µm, WC=57.25). (Invention)
7. Ultra- drivalon 250-4000 µm (PVA particles). (Reference)

Irinotecan hydrochloride trihydrate (Campto, from Aventis), was used at a concentration of 20 mg/ml. Other ingredients within this formulation include sorbitol and lactic acid. The concentration of camptothecin compound was determined using UV spectroscopy at 369 nm.

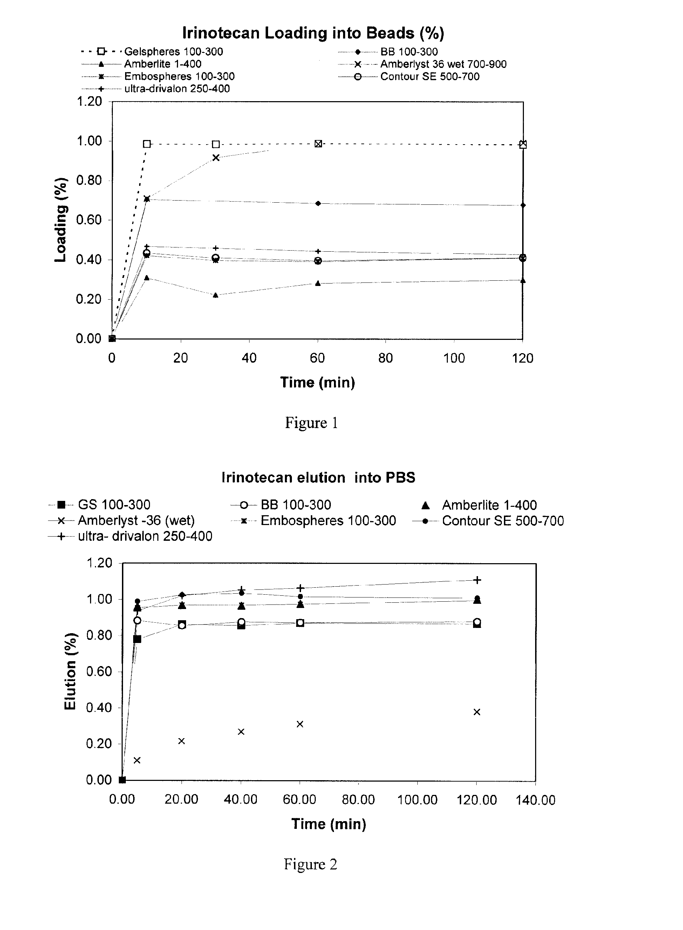

1 ml of each Bead slurry was mixed with 1ml of irinotecan solution (20 mg/ml) in a calculated amount, rotating-mixed for 2 hours at room temperature. The solution concentration remaining was measured with UV at 369 nm to determine the irinotecan concentration. The amount of drug loaded into the beads was calculated by depletion method. Figure 1 shows the loading characteristics of the microspheres under study. Clearly the beads with ionic components are able to load appreciable amounts of the drug (GelSpheres and Amberlyst particularly). Loading is particularly rapid for GelSpheres (5-10 mins) whereas the Amberlyst requires ∼60 mins.

These beads actively load the entire 20mg concentration of the drug from solution. The other embolic agents are only capable of loading 5-7 mg from the solution, which is essentially an equilibrium partitioning effect, indicating no specific interaction between bead and drug. Irinotecan was eluted from 1 ml of loaded beads as described above into 200ml of PBS buffer, at room temperature for 2 hours. Results (Figure 2) show almost the same elution rate for all beads, with an elution of more than 90% of the total eluted in the first 10 minutes. And complete within 2 hours, with the exception of amberlyst36 wet, which shows a slower elution profile, with 40% eluted in the first 2 hours. This is attributed to the high level of strongly acidic sulphonic acid component.

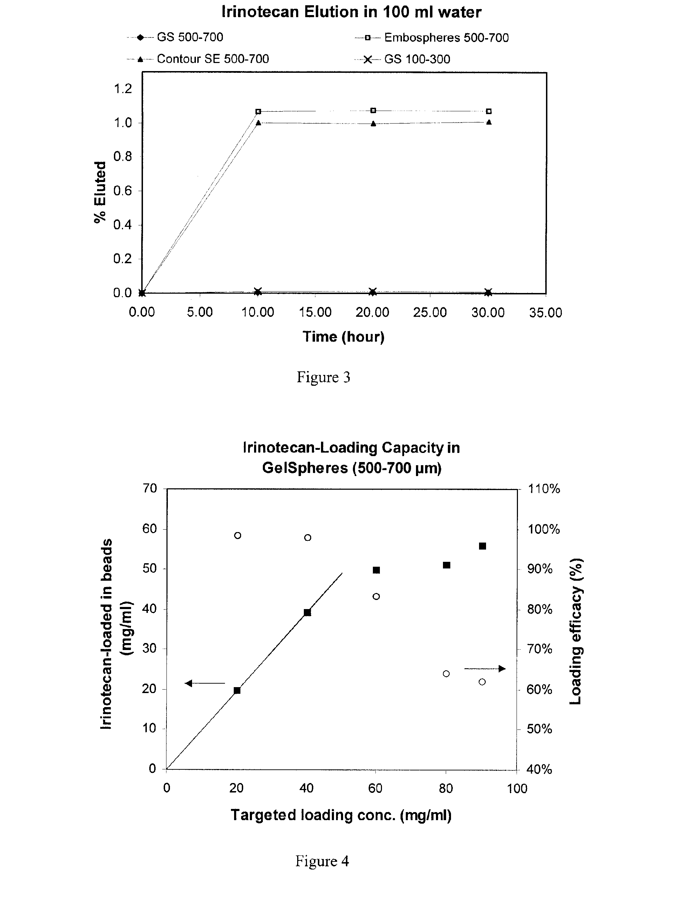

Figure 3 however shows the comparison of the elution profiles of different irinotecan loaded beads into water. 1 ml of loaded beads was eluted into 100 ml of water (HPLC grade) for 30 minutes. Contour SE and Embospheres beads show 100% elution within the first 10 minutes whereas GelSphere beads show an elution of less than 1 % of the total loaded. This indicated that elution is driven by an ion exchange mechanism and suggests that it will be possible to formulate the spheres of the invention in a hydrated form within pure water without fear of loss of the drug by elution into the media over time during storage. This feature would not be possible with the current commercial microspherical embolic agents.

### Example 2: Investigation of GelSpheres Loading Capacity

Irinotecan-loading content and loading efficacy was determined using GelSpheres, 500-700 µm. A bead slurry was mixed with irinotecan solution (20 mg/ml) in calculated amount, rotating-mixed for at least 4 hours. The solution was measured with UV at 369 nm to determine the irinotecan concentration and the drug-loading in beads (by depletion method). The straight line in Figure 4 shows that irinotecan content in beads linearly increased with designed loading amount under low concentration (below 50 mg/ml). Above this the loading efficacy dropped remarkably, indicating saturation of the beads.

### Example 3: Size Change with Irinotecan Loading

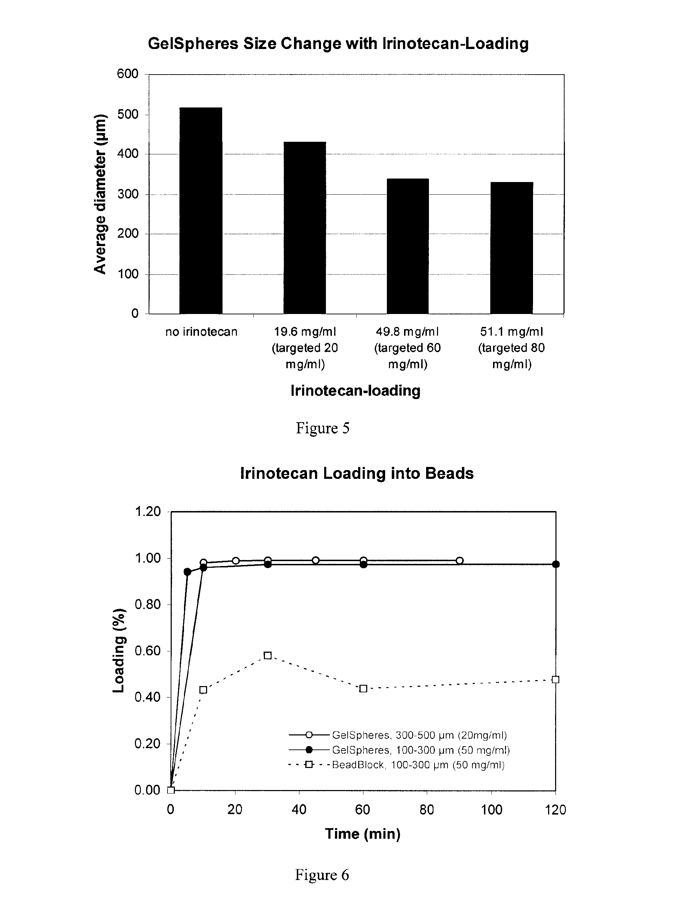

The GelSpheres size change with irinotecan-loading was measured by use of Image-ProPlus 4.5 with optical video microscopy. The loading condition is GelSpheres size, 500-700 µm; the concentration of irinotecan loading solution is 20 mg/ml (Campto) at room temperature with overnight on a roller mixer. Figure 5 shows there is a decrease in bead size with increasing concentration of drug associated within the beads. This is associated with displacement of water from the hydrogel structure by the drug interacting with the ionic groups.

### Example 4: Effect of Bead Size and Ionic Group Content on Drug Loading

A comparison of irinotecan-loading rate into high-AMPS GelSpheres of different sizes and low-AMPS BeadBlock. Loading conditions were 1 ml of each bead slurry (100-300 µm GelSpheres and BeadBlock) was mixed with 2.5 ml irinotecan solution (20 mg/ml); 1 ml of bead slurry (300-500 µm GelSpheres) was mixed with 1 ml irinotecan solution (20 mg/ml). The mixtures were rotating-mixed and the solution concentration was measured with UV at 369 nm. Figure 6 shows GelSpheres of different sizes load at similar, very rapid rates; low-AMPS spheres load less drug due to a lower concentration of ionic component of the microspheres.

### Example 5: Lyophilisation of Irinotecan-Loaded GelSpheres

1 ml of GelSphere beads was mixed with Campto (20mg/ml) solution and roller-mixed for 3 hours. The remaining solution was removed using a pipette to leave a bead slurry that was lyophilised to a dry product. Different loading levels are achieved by varying the amount of drug solution.

### Example 6: Elution from Irinotecan from Lyophilised GelSpheres

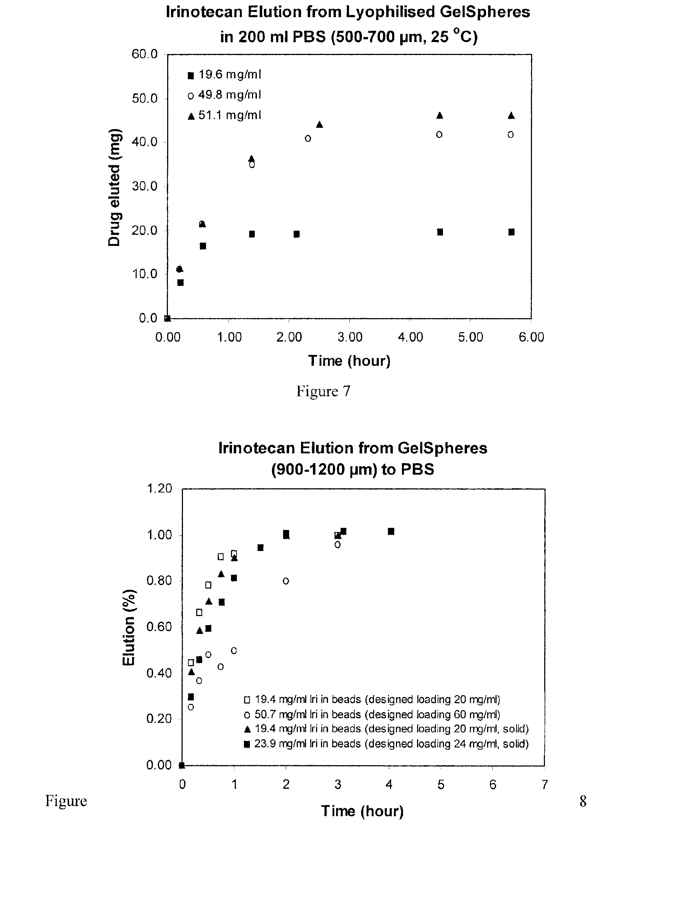

Irinotecan was eluted from lyophilised GelSpheres with different loadings of camptothecin as prepared in Example 5 into PBS buffer. The results are shown in Figure 7. The elution rate was slowed down after lyophilisation when compared with the non-lyophilised samples. Also the higher drug loading showed a slower elution compared to the lower one.

### Example 7: Comparison of Elution of Formulated and Non- formulated Irinotecan Hydrochloride

1 ml bead slurry was mixed with Campto formulation and roller-mixed for 3-4 hours. In a separate loading study 1 ml of beads were loaded with solid irinotecan hydrochloride neat drug by mixing the bead slurry with the powdered drug and 2 ml water, and roller-mixed for 3-4 hours over which time the drug dissolved slowly and was actively taken into the beads. The Irinotecan was eluted from the various 900-1200 µm GelSpheres into 200 ml PBS buffer. Elution curves shown in Figure 8 show no significant difference between the beads loaded from formulation or those loaded using the neat drug.

### Example 8: Drug Loading Indication

GelSpheres microspheres are tinted blue using Reactive Blue 4 dye in order that they can be easily visualised by interventional radiologists during use. The microspheres possess a blue colouration that is seen to shift to a turquoise colour upon loading of the irinotecan into the beads. This can be used as a visual indicator to differentiate between loaded and unloaded beads. The change in colouration is even more distinct in lyophilised irinotecan-loaded beads.

### Example 9:

### Summary of preclinical pilot study of irinotecan- and doxorubicin-loaded GelSpheres in the CC531-IacZ rat liver metastasis model

The purpose of this pilot study was to evaluate the effectiveness of drug eluting beads for chemoembolisation in a rat liver metastasis model, using irinotecan-loaded beads or doxorubicin-loaded beads. The objectives of this study were to assess the feasibility, to determine the reduction in tumour burden in rats treated with chemoembolisation, and to determine the dose of drug to be used in the main study.

The rat model was chosen for this study as a suitable model for chemoembolisation as it was previously demonstrated using this model that there was significant activity of irinotecan in terms of complete remission in 44% of rats and reduction of the mean tumour cell load by 66%. (*Saenger et al., op. cit*). In this model CC531-lacZ cells are transplanted by portal vein injection into male WAG/Rij rats and detection of tumour cells is 5 accomplished by their β-galactosidase activity. This allows the determination of the number of cells using a chemoluminescence assay.

Due to the small size of vessels in rats and in order to be consistent with earlier studies, the microspheres product with a size of 75 µm ± 25 µm will also be used. The microspheres will be made specifically for the study o by the method detailed previously in example 1 (high Amps) and tinted and sterilized as per normal procedures.

The drug will be mixed with the microspheres immediately prior to embolisation.

The drug and microspheres are left for 30 to 60 minutes to load and 5 agitated every 5 to 10 minutes to load. Alternatively they are placed on a rotary mixer to aid loading.

Tumour cells are injected into the portal vein of the rat model on day 0. A relaparatomy is performed on day 8 which allows a visual control of the presence of tumour cells in the liver. Animals found to be tumour positive will receive the embolisation treatment through the hepatic artery on day 8. On day 21 , the experiment is to be terminated. The liver weight of the animals will be determined and the livers deep frozen until the time when tumour cell number is to be determined by luminometry.

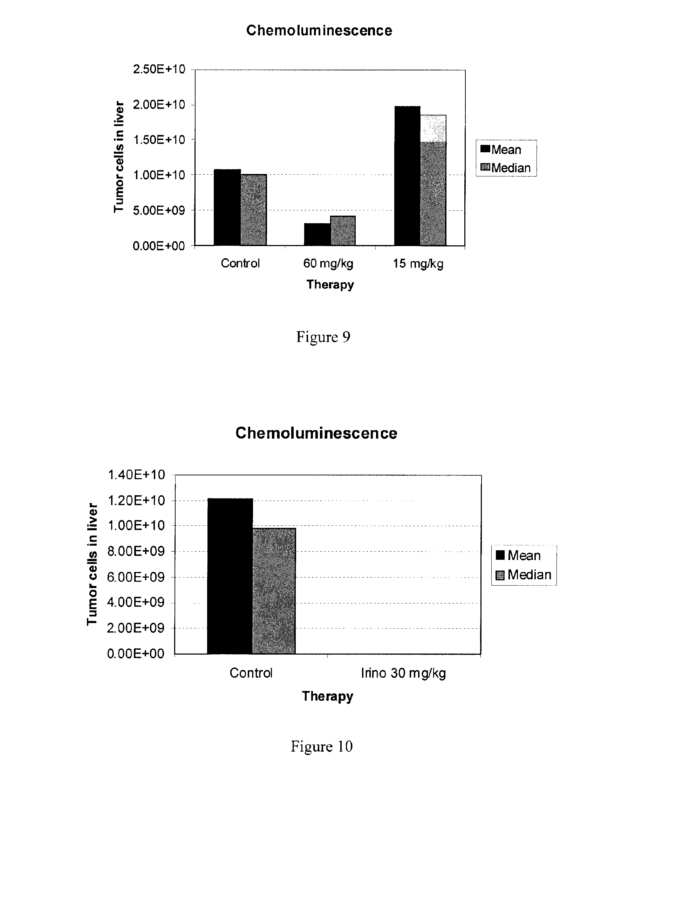

The following doses of irinotecan were used in the pilot study: 5 60mg/kg, 30mg/kg and 15mg/kg. The results are shown in Figure 9 which shows mean and median number of viable tumour cells in liver for control (n=9), 60 mg/kg and 15mg/kg (n=3) irinotecan-loaded bead groups.

Using a chemoluminescence assay, the number of viable tumour cells in the liver was measured in control animals and in test animals. Figure 9 o shows that there is more than a two-fold reduction in the number of viable tumour cells in rats that underwent chemoembolisation at a dose of irinotecan of 60 mg/kg. Although the 15mg/kg group appears to have a higher number of tumour cells, it should be noted that in the control group a number of the tumour cells have become necrotic, whereas in the 15mg/kg group the growth rate of the tumour was slower and as a results necrosis is lower leading to a higher number of viable tumour cells at this time point.

An additional experiment with 30 mg/kg of irinotecan showed complete disappearance of tumour cells in rat liver (figure 10). Figure 10 shows the mean and median numbers of tumour cells in liver for the control and 30 mg/kg irinotecan groups.

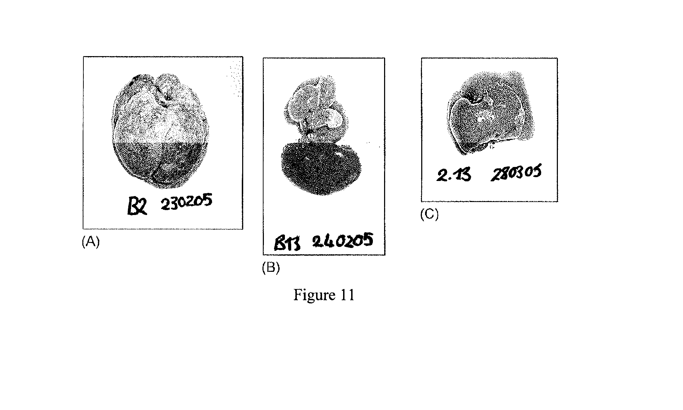

Figure 11 shows the appearance of the liver at the time of sacrifice. The control liver (A) shows the diffuse appearance of the tumour throughout the liver, as well as an increased in volume of the liver. The liver from animals after chemoembolisation with 60 (B) or 30 (C) mg/kg of irinotecan shows that the liver has an apparently healthy appearance with no detectable tumour and no increase in liver volume.

## Claims

1. A composition comprising microspheres comprising a water-insoluble water-swellable polymer which is anionically charged at pH7 and electrostatically associated with the polymer in releasable form, a cationically charged camptothecin compound in which the microspheres have diameters when equilibrated with water at room temperature of more than 100µm.

2. A composition according to claim 1 in which the camptothecin compound has the general formula I 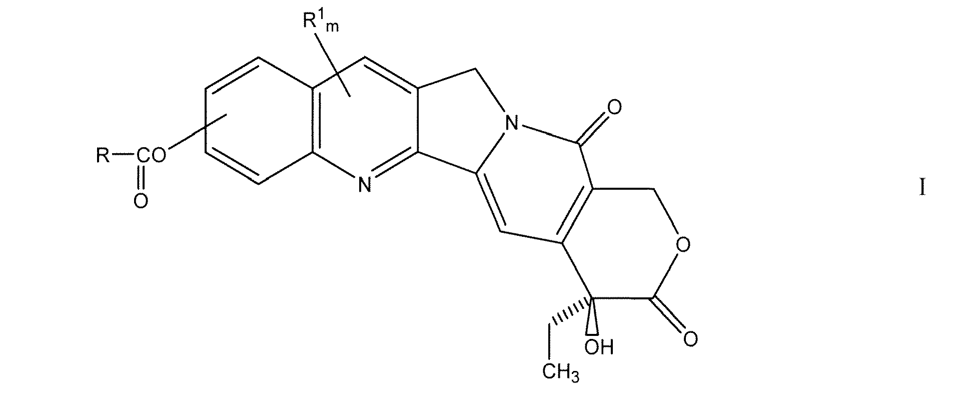
in which R¹ is H, lower (C₁₋₆) alkyl, optionally substituted by a hydroxyl amine, alkoxy, halogen, acyl or acyloxy group or halogen; and
R is chlorine or NR²R³ where R² and R³ are the same or different and each represents a hydrogen atom, a substituted or unsubstituted C₁₋₄ alkyl group or a substituted or unsubstituted carbocyclic or heterocyclic group, or R² and R³ together with the nitrogen atom to which they are attached from a optionally substituted heterocyclic ring which may be interrupted by -O-, -S- or >NR⁴ in which R⁴ is a hydrogen atom, a substituted or unsubstituted C₁₋₄ alkyl group or a substituted or unsubstituted phenyl group;
and wherein the grouping -0-CO-R is bonded to a carbon atom located in any of the 9, 10 or 11 positions in the A ring of the camptothecin compound, including salts thereof.

3. A composition according to claim 2 in which R is NR²R³ in which R² and R³ together with the nitrogen atom form a optionally substituted heterocyclic ring.

4. A composition according to claim 3 in which R is

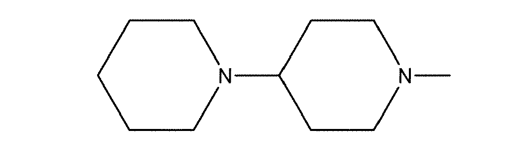

5. A composition according to any of claims 2 to 4 in which RCOO- is substituted at the 10 position.

6. A composition according to any of claims 2 to 5 in which R¹ is ethyl and m is 1.

7. A composition according to any preceding claim in which the microspheres have sizes when equilibrated in water at 37°C, in the range 100 to 1500 µm.

8. A composition according to any preceding claim in which the polymer is crosslinked polyvinylalcohol.

9. A composition according to claim 8 in which the polymer is formed from polyvinyl-alcohol macromer, having more than one ethylenically unsaturated pendant group per molecule, by radical polymerisation of the ethylenic groups.

10. A composition according to claim 9 in which the PVA macromer is copolymerised with ethylenically unsaturated monomer.

11. A composition according to claim 10 in which the monomer includes ionic monomer having the general formula II 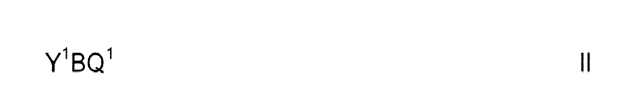 in which Y¹ is selected from 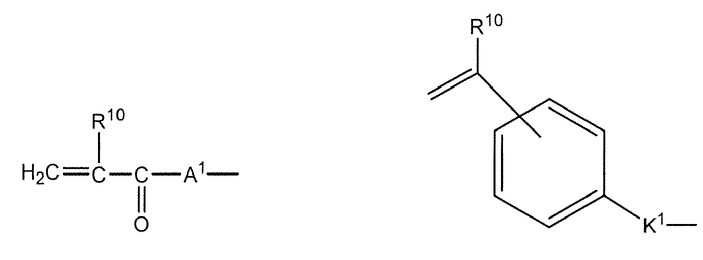 CH₂=C(R¹⁰)-CH₂-O-, CH₂=C(R¹⁰)-CH₂ OC(O)-, CH₂=C(R¹⁰)OC(O)-, CH₂=C(R¹⁰)-O-, CH₂=C(R¹⁰)CH₂OC(O)N(R¹¹)-, R¹²OOCCR¹⁰=CR¹⁰C(O)-O-, R¹⁰CH=CHC(O)O-, R¹⁰CH=C(COOR¹²)CH₂-C(O)-O-, 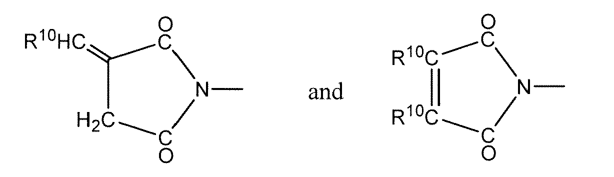 wherein:
R¹⁰ is hydrogen or a C₁-C₄ alkyl group;
R¹¹ is hydrogen or a C₁-C₄ alkyl group;
R¹² is hydrogen or a C₁₋₄ alkyl group or BQ¹ where B and Q¹ are as defined below;
A¹ is -O- or -NR¹¹ -;
K¹ is a group -(CH₂)ᵣOC(O)-, -(CH₂)ᵣC(O)O-, -(CH₂)ᵣOC(O)O-, -(CH₂)ᵣNR¹³-, -(CH₂)ᵣNR¹³C(O)-, -(CH₂)ᵣC(O)NR¹³-, -(CH₂)ᵣNR¹³C(O)O-, -(CH₂)ᵣOC(O)NR¹³-, - (CH₂)ᵣNR¹³C(O)NR¹³- (in which the groups R¹³ are the same or different), -(CH₂)ᵣO--(CH₂)ᵣSO₃ -, or, optionally in combination with B, a valence bond and r is from 1 to 12 and R¹³ is hydrogen or a C₁-C₄ alkyl group;
B is a straight or branched alkanediyl, oxaalkylene, alkanediyloxaalkanediyl, or alkanediyloligo(oxaalkanediyl) chain optionally containing one or more fluorine atoms up to and including perfluorinated chains or, if Q¹ or Y¹ contains a terminal carbon atom bonded to B a valence bond; and
Q¹ is an anionic group.

12. A composition according to claim 11 in which Q¹ is a carboxylate, carbonate, sulphonate, sulphate, nitrate, phosphonate or phosphate group.

13. A composition according to claim 11 or 12 in which Y¹ is a group CH₂=CR¹⁰COA- in which R¹⁰ is H or methyl, preferably methyl, and in which A¹ is preferably NH and B is an alkanediyl group of 2 to 6 carbon atoms.

14. A composition according to any preceding claim which additionally comprises a liquid suspending agent.

15. A composition according to any preceding claim which comprises an imaging agent preferably a radiopaque imaging agent.

16. A composition according to any of claims 1 to 14 which is in substantially dry particulate form, preferably lyophilised form.

17. A composition according to claim 1 wherein the polymer is swellable so as to have equilibrium water content when swollen in water at 37°C, measured by gravimetric analysis, in the range 40 to 99% by weight, preferably 75 to 95%.

18. A composition according to claim 1 wherein the camptothecin compound has the general formula III 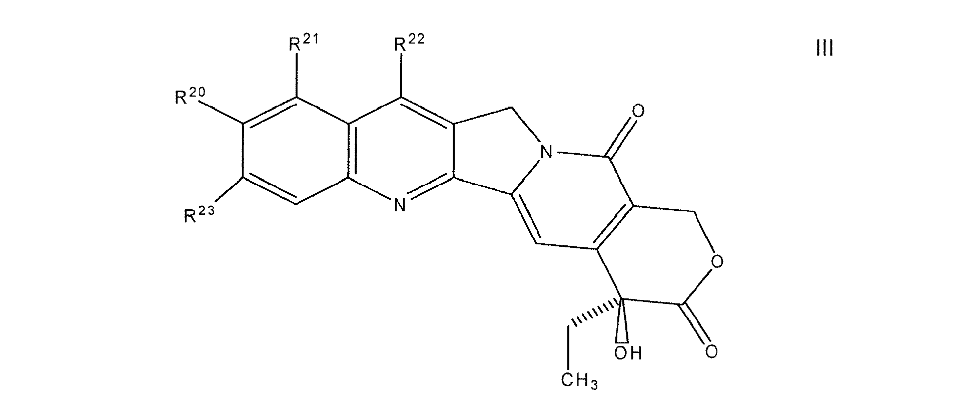
in which R²⁰ and R²³ are each hydroxy or hydrogen or together are CH₂OCH₂;
one of R²¹ and R²² is H and the other is CH₂NR²⁴R²⁵ where R²³ and R²⁴ are the same or different and each represents a hydrogen atom, a substituted or unsubstituted C₁₋₄ alkyl group or a substituted or unsubstituted carbocyclic or heterocyclic group, or R²³ and R²⁴ together with the nitrogen atom to which they are attached from a optionally substituted heterocyclic ring which may be interrupted by -O-, -S- or >NR⁴ in which R⁴ is a hydrogen atom, a substituted or unsubstituted C₁₋₄ alkyl group or a substituted or unsubstituted phenyl group; including salts and quaternary derivatives thereof.

19. A composition according to claim 18 wherein the camptothecin compound is topotecan.

20. A composition according to any preceding claim for use in a method of treatment of a solid tumour.
